# FASCICULE DE BREVET EUROPEEN

(11) **EP 2 752 651 B1**
(45) Date de publication et mention de la délivrance du brevet: **17.02.2016**
(21) Numéro de dépôt: 13198962.6
(22) Date de dépôt: 20.12.2013
(51) Int. Cl.: G01N 5/04, F26B 21/00, G01N 33/02

(54) **Analyseur d'humidité et procédé d'analyse d'humidité d'échantillon de végétaux**
Vorrichtung und Verfahren zur Analyse des Feuchtegehaltes einer pflanzlichen Probe
Device and method for analysing the humidity of a plant sample

(30) Priorité: 07.01.2013 FR 1350113
(43) Date de publication de la demande: 09.07.2014
(73) Titulaire: FLORETTE Holding, 50430 Lessay (FR)
(72) Inventeur: Savary, Laurent, 50430 Angoville sur Ay (FR)
(74) Mandataire: Cabinet HERRBURGER

(56) Documents cités:
- US-A- 4 777 734
- US-A- 4 964 734
- US-A- 5 318 049
- US-A1- 2003 030 450

## Description

### Domaine de l'invention

La présente invention se rapporte à un analyseur d'humidité d'échantillons de végétaux ainsi qu'un procédé d'analyse d'humidité appliqué par un tel analyseur.

Dans le cadre de la description de l'invention, les échantillons de végétaux sont au sens général des feuilles de végétaux telles que des feuilles de salade ou des feuilles de légumes, des morceaux de légumes ou de fruits, voire de légumes et de fruits entiers dont on veut déterminer l'humidité en surface, par exemple dans le cadre de contrôles industriels d'un procédé.

### Etat de la technique

Actuellement pour déterminer l'humidité résiduelle de feuilles de végétaux par exemple avant leur conditionnement en sachet en sortie d'un procédé de nettoyage et de traitement, on procède d'abord à la pesée d'un échantillon de produit à contrôler, puis l'essorage de l'échantillon pendant un temps donné et à une vitesse définie et ensuite à la pesée de l'échantillon essoré. Le taux d'humidité résiduelle s'obtient par la différence du poids entre l'échantillon initial et l'échantillon essoré.

Ce procédé ne prend pas en compte toute l'humidité résiduelle sur le produit, car l'essorage par centrifugation élimine uniquement les gouttelettes d'eau, alors que la pellicule superficielle d'eau reste sur les feuilles de végétaux, notamment les feuilles de salade.

On connaît également un autre procédé consistant à sécher les feuilles avec un buvard. Les feuilles sont essuyées une à une pour absorber l'eau à la surface des feuilles et déterminer ainsi l'humidité résiduelle. Ce procédé est fiable mais il se fait manuellement et demande beaucoup de temps au niveau du poste de contrôle d'une fabrication.

Il existe également des analyseurs d'humidité fonctionnant par infrarouge, par halogène ou laser. Ils sont destinés à contrôler des produits ayant une faible teneur en humidité et qui est régulière sur l'ensemble du produit. Mais ces analyseurs d'humidité ne conviennent pas pour des feuilles de végétaux telles que des feuilles de salade pour lesquelles l'humidité résiduelle peut aller jusqu'à 20 % et surtout elle est irrégulière sur toute la surface de la feuille.

Il existe également un document US 2003/0030450 décrivant un procédé et un appareil d'essai pour déterminer l'état de surface sec d'un agrégat, c'est-à-dire d'une masse de sable et de pierre. L'appareil mettant en oeuvre le procédé se compose d'un caisson 10 regroupant tout l'équipement de l'appareil. Le caisson est formé d'un boitier 12 ayant une chambre à échantillon 14 logeant un tambour 24 recevant l'échantillon à analyser. L'équipement est complété par une entrée et une sortie d'air mis en circulation par un ventilateur 38. Le procédé a pour but de déterminer le poids à sec de l'échantillon à partir d'un état sec superficiel (état SSD). Pour déterminer le poids final de l'échantillon, après avoir déterminé la tare constituée par l'appareil vide placé sur une balance 60, on charge l'appareil avec l'échantillon d'agglomérat et on le met en oeuvre en faisant circuler l'air chaud à travers l'échantillon tournant dans le tambour. Des capteurs d'humidité de l'air permettent de déterminer le point SSD et partant de là, de déterminer l'état complètement sec de l'agglomérat, c'est-à-dire lorsque celui-ci ne contient plus aucune humidité. L'ensemble est surveillé par des capteurs d'humidité. Le procédé s'exécute par une pesée en continu sur la balance 60. Cela signifie que la balance porte le caisson 10 avec ses équipements de circulation et de chauffage d'air, mis en mouvement ou tournant pendant la pesée ainsi que l'entraînement en rotation du tambour 24 chargé de l'échantillon d'agrégat. Cela soumet la balance à un traitement brutal, incompatible avec une pesée de précision.

Dans le cas de ce document, la pesée est possible, même d'une façon archaïque, car la masse de l'échantillon d'agrégat est en relation avec la masse de l'appareil, l'ensemble étant posé sur la balance. Mais un tel équipement ne permettrait pas une pesée différentielle d'échantillons même représentatifs de quelques dizaines de grammes seulement.

### But de l'invention

La présente invention a pour but de développer un analyseur d'humidité et son procédé de mise en oeuvre permettant de déterminer l'humidité en surface de végétaux telles que des feuilles de salade ou autres feuilles, des morceaux de légumes, de fruits ou des légumes ou fruits entiers, notamment à surface irrégulière et destinées à être conditionnées, notamment pour utiliser les informations pour la gestion d'un procédé de préparation de feuilles de végétaux comprenant une étape de séchage, de façon à permettre par des moyens simples et rapides de déterminer efficacement et précisément l'humidité résiduelle de feuilles de végétaux.

### Exposé et avantages de l'invention

A cet effet, l'invention a pour objet un analyseur d'humidité d'échantillons de végétaux, comprenant :
- un récepteur recevant la charge formée d'un échantillon de végétaux, et entraîné en rotation par un dispositif d'entraînement,
- une installation de séchage par air sec mis en température ayant des organes de soufflage dirigés sur la charge à travers le récepteur,
- une installation de pesée du récepteur contenant la charge,
- une unité de commande reliée au récepteur au dispositif d'entraînement en rotation à l'installation de séchage et à l'installation de pesée pour commander la pesée du récepteur chargé avant et après la phase de séchage ainsi que la mise en rotation du récepteur et l'actionnement de l'installation de séchage,
- le récepteur est monté en rotation dans un support porté par une unité de positionnement mettant le récepteur en prise avec le dispositif d'entraînement pour l'entraîner en rotation ou le dégager avant et après la phase d'entraînement en rotation et le mettre dans l'installation de pesée.

Cet analyseur d'humidité permet d'analyser des échantillons de végétaux tels que des feuilles de salade dont l'humidité résiduelle peut être très élevée et comprise entre 0 et 20 %. L'analyseur est particulièrement efficace malgré la surface irrégulière des feuilles analysées. La précision de l'analyse faite par cet analyseur est particulièrement poussée, car les produits analysés ne sont pas manipulés en cours d'analyse. Ils sont placés dans le récepteur pour subir les opérations de séchage et de pesée avant et après séchage.

L'air sec de l'installation de séchage est de préférence un air ultra sec ayant entre 0 et 5 % d'humidité à 25°C et mis en température de préférence à une température de 25°C de façon à favoriser efficacement le séchage sans détériorer les caractéristiques du produit analysé et surtout sans sécher le produit lui-même, l'opération de séchage se limitant à l'enlèvement de l'humidité superficielle des produits (échantillons de végétaux).

Grâce à la mise en rotation du récepteur, tous les échantillons sont régulièrement exposés au flux d'air sec ce qui garantit l'homogénéité du séchage. L'efficacité du séchage permet d'analyser une charge significative, par exemple entre 25 et 30 grammes, de sorte que le résultat de l'analyse est très représentatif des produits séchés.

Les mesures faites grâce à cet analyseur sont particulièrement fiables car alors que le procédé actuel de séchage, tel que décrit ci-dessus, donne par exemple une humidité résiduelle de seulement 2 %, alors que l'analyseur d'humidité selon l'invention donne une humidité de 8 % beaucoup plus proche de l'état réel.

Suivant une autre caractéristique avantageuse, le récepteur comprend un panier ajouré, perméable aux jets d'air des buses, monté en rotation dans le support porté par une unité de positionnement mettant le panier en prise avec le dispositif d'entraînement pour l'entraîner en rotation ou le dégager et le mettre dans l'installation de pesée.

Cette forme de réalisation du récepteur comme panier ajouré est intéressante pour sécher efficacement les échantillons de végétaux malgré la charge relativement importante utilisée pour qu'elle soit représentative et significative.

De façon avantageuse, l'installation de séchage par air sec mis en température est constituée par une source d'air sec sous pression, alimentant un distributeur pour sa mise en température et sa distribution selon un mode de distribution avec des buses dirigées sur la charge dans différentes directions.

L'orientation différente des axes de soufflage permet d'agiter et de traiter les feuilles sous des angles différents ce qui évite que les rugosités et les formes irrégulières des échantillons ne retiennent de l'eau prisonnière dans des zones en contre-dépouille par rapport à telle ou telle direction de soufflage.

D'une manière particulièrement avantageuse et simple, l'une au moins des buses a son axe de soufflage dirigé radialement dans un plan sensiblement perpendiculaire à l'axe de rotation du panier et l'autre buse a son axe de soufflage dirigé sensiblement parallèlement à l'axe de rotation.

Suivant une caractéristique avantageuse, l'analyseur comporte une enceinte dans laquelle sont logés le récepteur et au moins les buses de l'installation de séchage ainsi que l'installation de pesée, de sorte que l'analyseur constitue un ensemble compact, transportable, les équipements tels que le distributeur relié à la source d'air comprimé ou l'unité de commande, le moyen d'enregistrement et d'affichage et le clavier, étant situés à l'extérieur de l'enceinte sur le boîtier de l'analyseur.

Suivant une autre caractéristique avantageuse, l'enceinte comporte un extracteur d'air évacuant l'air humide de l'enceinte ce qui favorise l'efficacité du séchage.

Suivant une autre caractéristique avantageuse, l'unité centrale comporte un moyen d'enregistrement et d'affichage des informations d'analyse et un moyen d'entrée des paramètres. Ce moyen d'enregistrement et d'affichage permet de stocker les données de l'analyse et de les afficher ou encore de les transmettre à un système central par une liaison radio par l'intermédiaire d'une interface.

Le moyen d'entrée des paramètres est de préférence un clavier pour gérer les paramètres et commander la mise en marche et plus généralement le fonctionnement de l'analyseur d'humidité en étant relié à l'unité centrale.

Suivant une autre caractéristique avantageuse, l'unité de positionnement du récepteur par rapport au dispositif d'entraînement en rotation et à l'installation de pesée, est un organe de soulèvement relié au récepteur pour les opérations de positionnement, l'installation de pesée étant située sous le récepteur pour que le dispositif de positionnement puisse procéder par simple mouvement de soulèvement et d'abaissement.

Suivant une autre caractéristique avantageuse, le dispositif d'entraînement en rotation comporte un moteur et une roue d'entraînement qui coopère directement avec le panier en roulant sur le côté du panier ou sur l'axe de rotation du panier.

En résumé, l'analyseur d'humidité constitue un dispositif particulièrement simple et efficace d'analyse rapide de l'humidité résiduelle d'échantillons de végétaux tels que des feuilles de salade, s'intégrant efficacement dans une ligne de production pour contrôler et gérer le fonctionnement de la ligne de production du moins pour la partie concernant le séchage des produits.

### Dessin

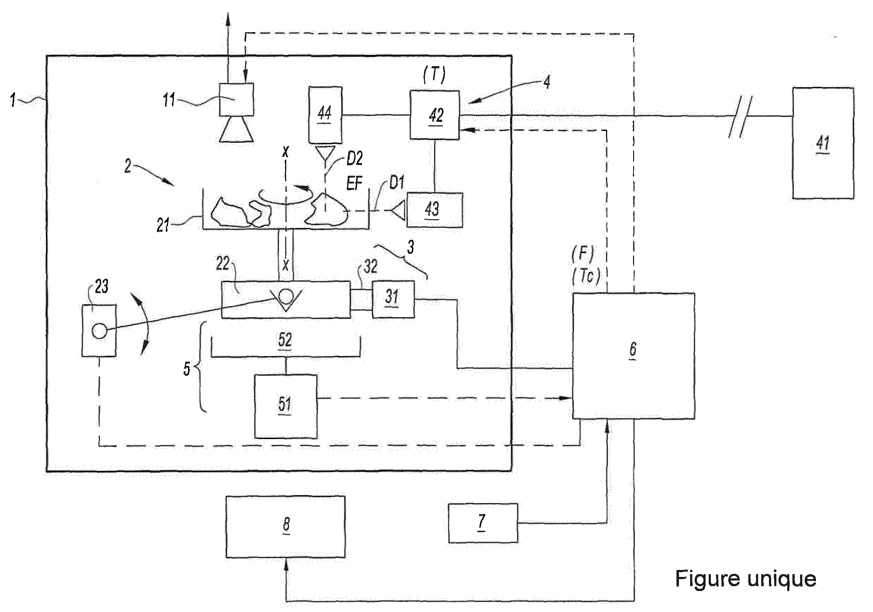

La présente invention sera décrite ci-après à l'aide d'un exemple d'analyseur d'humidité représenté schématiquement dans le dessin dans lequel l'unique figure est un schéma par blocs de l'analyseur d'humidité.

### Description d'un mode de réalisation de l'invention

Selon la figure, l'invention a pour objet un analyseur d'humidité d'échantillons de végétaux, notamment d'un échantillon représentatif de feuilles (EF) telles que des feuilles de salade dont on veut mesurer l'humidité résiduelle après un séchage, par exemple dans le cadre un procédé de préparation et de conditionnement de feuilles de végétaux tels que des feuilles de salade ou des morceaux de végétaux.

Cette analyse permet de déterminer l'état de séchage (c'est-à-dire l'enlèvement de l'humidité superficielle) de végétaux sortant d'un traitement par voie humide suivi d'un séchage, en vue de leur conditionnement par exemple des feuilles. Cette analyse peut s'appliquer aussi à différentes phases intermédiaires d'un procédé industriel pour déterminer à chaque étape, l'humidité résiduelle des végétaux et par exemple l'efficacité du séchage en fonction des différents types de végétaux. Cette opération a pour but de déterminer l'humidité résiduelle et non pas la teneur en eau des végétaux.

L'analyseur d'humidité se compose d'une enceinte 1 logeant un récepteur 2 monté sur un dispositif d'entraînement en rotation 3 et recevant une charge sous la forme d'un échantillon de végétaux tels que des feuilles représentatif (EF) à analyser ainsi que d'une installation de séchage par air sec 4, d'une installation de pesée 5 et d'une unité de commande 6. Cette dernière est reliée au récepteur 2, au dispositif d'entraînement 3, à l'installation de séchage 4, à l'installation de pesée 5 pour gérer le fonctionnement et les étapes d'une analyse automatique en fonction de paramètres introduits dans l'analyseur, notamment par un clavier 7 et un moyen d'enregistrement et d'affichage 8 fournissant les résultats directement ou les exportant par une liaison avec l'interface.

Le récepteur 2 comporte un récipient ajouré en forme de panier 21 monté à rotation dans un support 22 de façon à tourner autour de son axe XX en étant mis en rotation par le dispositif d'entraînement 3 dont le moteur 31 est en prise par sa roue d'entraînement 32 avec le panier 21 ou son support 22. Le dispositif d'entraînement 3 est de préférence dégagé du récepteur 2 avant et après la phase d'entraînement en rotation. La roue 32 est au contact par exemple de la périphérie du panier ou d'un chemin de roulement sur le panier 21. Le panier n'étant pas une centrifugeuse, son entraînement en rotation se fait à vitesse relativement réduite pour ne pas abîmer les produits tout en les déplaçant par rapport aux axes de soufflage D1, D2 des buses pour présenter aussi diversement que possible les échantillons tels que les feuilles (EF) des produits à l'action de l'air sec de séchage. Le panier 21 a une structure formée de fils en acier inoxydable ou en matière plastique ; cette structure est suffisamment fine pour ne pas accrocher l'humidité et permettre une excellente ventilation de la charge (EF).

L'installation de séchage 4 se compose d'une source d'air sec 41 voire très sec, par exemple le réseau d'air comprimé de l'entreprise ou une source d'air comprimé, indépendante, fournissant de l'air comprimé sec. La source d'air comprimé 41 est reliée par un distributeur 42 à un ensemble de buses 43, 44 dirigées sur la charge (EF) du récipient 21. De façon préférentielle, une ou plusieurs buses 43 sont réparties avec leur axe de soufflage D1 dans un plan sensiblement perpendiculaire à l'axe XX de rotation du panier 21 ou suivant des directions légèrement inclinées par rapport à un tel plan passant par le panier et notamment dans la direction radiale. Une autre partie de l'ensemble des buses 44 et au moins l'une de celles-ci est orientée avec son axe D2 descendant en direction du panier 21 mais de façon à ce que ce ou ces axes soient sensiblement parallèles à l'axe XX ou du moins coupent la direction des axes de soufflage D1 des buses 43 du premier ensemble.

De façon générale, les axes de soufflage D1, D2 des buses 43, 44 ne sont pas parallèles de façon à toucher la charge (EF) du panier 21 suivant différents angles pour la meilleure efficacité du séchage, c'est-à-dire l'enlèvement de l'humidité superficielle sans sécher les échantillons eux-mêmes.

L'air sec est soufflé par les buses 43, 44 sur les échantillons du panier 21 mis en rotation pour que les jets d'air sec atteignent toutes les surfaces des échantillons. Pour favoriser le séchage, l'air sec est mis de préférence à la température (T) la plus efficace pour chaque type de végétaux. Cette température (T) fixée comme température de consigne (TC) par exemple à 25°C dans l'unité de commande 6 est réglée par le distributeur 42. Dans le schéma, on a figuré deux exemples de buses 43, 44 dont l'axe de soufflage D1 de l'une est radial et perpendiculaire à l'axe XX. L'autre axe de soufflage D2 est parallèle à l'axe XX. Ces deux axes D1, D2 ne se coupent pas nécessairement, de sorte qu'ils ne sont pas dans le même plan, ce qui est avantageux pour l'efficacité du séchage, c'est-à-dire la rapidité de l'écoulement du flux d'air à la surface des végétaux (EF) sans risquer de dessécher les végétaux eux-mêmes.

Le récepteur mobile 2 coopère avec l'installation de pesée 5, de préférence avant et après la phase de séchage. L'installation de pesée 5 constituée d'appuis 52 en forme de plateau et d'une unité de pesée 51 est placée sous le support de rotation 22 du récepteur 2 et de son panier 21 de façon à pouvoir déposer le récepteur 2 sur le plateau 52 et en même temps le détacher du moyen d'entraînement en rotation 3 du panier 21. Cette désolidarisation a pour but de réduire la tare autant que possible et de ne pas perturber l'installation de pesée 5 par la rotation du récepteur 2 avec sa charge (EF). Dans ces conditions le dispositif d'entraînement 3 n'intervient pas comme tare dans la pesée.

L'air chargé d'humidité de l'enceinte 1 est évacué par un extracteur 11.

L'unité de commande 6 gère le fonctionnement du récepteur 2 et à son dispositif d'entraînement 3, l'unité de positionnement 23, l'installation de pesée 5 ainsi que l'installation de séchage 4 et le moyen d'affichage et d'enregistrement 8 ou de transmission de données et le clavier de commande 7. L'unité de commande 6 gère la phase d'analyse selon les paramètres introduits dans l'analyseur qui dépendent le cas échéant de la nature des végétaux analysés. Ces paramètres sont la vitesse de rotation du récepteur, la durée de mise en rotation, le mode de fonctionnement (F) du distributeur d'air 42 alimentant les buses 43, 44, de façon continue ou de manière séquentielle avec de l'air à température réglée et pendant une durée déterminée ainsi que le dispositif de positionnement pour engager et dégager le récepteur 2 de l'installation de pesée 5 et inversement dégager ou engager le dispositif d'entraînement en rotation 3.

L'analyse d'humidité peut se faire de manière répétée suivant un cycle programmé s'il s'agit toujours des mêmes produits. Les paramètres de fonctionnement tels que la vitesse de rotation (VR), la durée du séchage (DS), l'intensité des jets d'air sec (DI) et la température (T), sont introduits dans l'unité de commande 6 et qui seront utilisés en fonction de la nature des produits analysés.

L'analyse d'humidité consiste à déterminer aussi précisément que possible l'humidité superficielle chargeant les échantillons de végétaux. Pour cela, on prend un échantillon (EF) représentatif placé dans le panier 21 du récepteur qui est ensuite pesé avant sa mise en rotation ; puis le récepteur 2 est dégagé de l'installation de pesée 5 pour être entraîné en rotation et présenter les feuilles de végétaux à l'action des jets d'air sec émis par les buses 43, 44. Après le temps de séchage (DS) déterminé par exemple de manière expérimentale, le soufflage d'air sec est arrêté de même que la rotation du panier 21 qui est ensuite replacé sur l'installation de pesée 5 pour peser la charge séchée et déterminer par différence, la perte d'humidité pendant la phase de séchage.

Pour tenir compte de l'éventuelle humidité déposée sur le panier 21 et qui n'est pas prise en compte par la pesée après séchage, il est possible de compléter les mesures par une pesée du panier vide après enlèvement des échantillons séchés. La différence entre la pesée du panier après enlèvement des échantillons et du panier à vide et sec avant la mise en place des échantillons, permet de déterminer la masse d'eau déposée sur la structure du panier et qui s'ajoute à la masse d'eau évacuée des échantillons et obtenue comme différence entre les deux premières pesées.

Enfin, pour une plus grande précision, il est également possible de peser le récepteur et son panier sec avant de le charger avec les échantillons à analyser.

Après l'analyse, on sèche le panier 21 par exemple en faisant tourner le panier vide dans les jets d'air sec avant d'effectuer une autre analyse.

L'analyseur d'humidité selon l'invention permet de déterminer l'humidité résiduelle sur des échantillons de végétaux tels que des feuilles de salade à la fin d'un cycle de traitement dans une installation industrielle et avant le conditionnement des feuilles dans des sachets, par exemple pour régler l'efficacité du séchage des feuilles et pour ne pas conditionner des feuilles dont l'humidité résiduelle serait trop importante. Le résultat de l'analyse d'humidité permet ensuite d'adapter le fonctionnement de l'installation industrielle.

### NOMENCLATURE DES ELEMENTS PRINCIPAUX

- 1: enceinte
- 11: extracteur d'air humide
- 2: récepteur
- 21: panier
- 22: support
- 23: unité de positionnement
- 3: dispositif d'entraînement en rotation
- 31: moteur
- 32: roue d'entraînement
- 4: installation de séchage
- 41: source d'air comprimé sec
- 42: distributeur d'air comprimé mis en température
- 43: buse de soufflage
- 44: buse de soufflage
- 5: installation de pesée
- 51: unité de pesée
- 52: plateau/appui
- 6: unité de commande
- 7: clavier/entrée de données
- 8: moyen d'enregistrement et d'affichage des résultats de pesée

- F: mode de fonctionnement

## Revendications

1. Analyseur d'humidité d'échantillons de végétaux, comprenant :
- un récepteur (2) recevant la charge formée d'un échantillon de végétaux (EF), et entraîné en rotation par un dispositif d'entraînement (3),
- une installation de séchage (4) par air sec mis en température ayant des organes de soufflage (43, 44) dirigés sur la charge (EF) à travers le récepteur (2),
- une installation de pesée (5) du récepteur (2) contenant la charge (EF),
- une unité de commande (6) reliée au récepteur (2) au dispositif d'entraînement en rotation (3), à l'installation de séchage (4) et à l'installation de pesée (5) pour commander la pesée du récepteur (2) chargé avant et après la phase de séchage ainsi que la mise en rotation du récepteur (2) et l'actionnement de l'installation de séchage (4),
**caractérisé en ce que**
- le récepteur (2) est monté en rotation dans un support (22) porté par une unité de positionnement (23) mettant le récepteur (2) en prise avec le dispositif d'entraînement (3) pour l'entraîner en rotation ou le dégager avant et après la phase d'entraînement en rotation et le mettre dans l'installation de pesée (5).

2. Analyseur d'humidité selon la revendication 1,
**caractérisé en ce que**
le récepteur (2) comprend un panier (21) ajouré perméable aux jets d'air des buses (43, 44).

3. Analyseur d'humidité selon la revendication 1,
**caractérisé en ce que**
l'unité de positionnement du récepteur par rapport au dispositif d'entraînement en rotation et à l'installation de pesée, est un organe de soulèvement relié au récepteur pour les opérations de positionnement, l'installation de pesée étant située sous le récepteur pour que le dispositif de positionnement puisse procéder par simple mouvement de soulèvement et d'abaissement.

4. Analyseur d'humidité selon la revendication 1,
**caractérisé en ce que**
le dispositif d'entraînement en rotation comporte un moteur et une roue d'entraînement qui coopère directement avec le panier en roulant sur le côté du panier ou sur l'axe de rotation du panier.

5. Analyseur d'humidité selon la revendication 1,
**caractérisé en ce que**
l'installation de séchage (4) par air sec mis en température, est constituée par une source d'air sec sous pression (41), réglée, alimentant un distributeur (42) pour sa mise en température (T') et sa distribution selon un mode de distribution (F) avec des buses (43, 44) dirigées sur la charge (EF) dans différentes directions (D1, D2).

6. Analyseur d'humidité selon la revendication 5,
**caractérisé en ce que**
l'une au moins des buses (43) a son axe de soufflage (D1) dirigé radialement dans un plan sensiblement perpendiculaire à l'axe (XX) de rotation du panier (21) et l'autre buse (44) a son axe de soufflage (D2) dirigé sensiblement parallèlement à l'axe de rotation (XX).

7. Analyseur d'humidité selon la revendication 1,
**caractérisé en ce qu'**
il comporte une enceinte (1) dans laquelle sont logés le récepteur (2) et au moins les buses (43, 44) de l'installation de séchage (4) ainsi que l'installation de pesée (5), l'enceinte (4) comporte notamment un extracteur d'air (11) évacuant l'air humide de l'enceinte (1),

8. Analyseur d'humidité selon la revendication 1,
**caractérisé en ce que**
l'unité centrale (6) comporte un moyen d'enregistrement et d'affichage (8) des informations d'analyse et un moyen d'entrée des paramètres (7).

9. Analyseur d'humidité selon la revendication 1,
**caractérisé en ce que**
l'installation de séchage fournit un air ultra-sec ayant entre 0 et 5 % d'humidité à 25°C.

10. Analyseur d'humidité selon la revendication 1,
**caractérisé en ce que**
l'installation de séchage fournit de l'air sec mis à une température réglée de 25°C.

## Patentansprüche

1. Feuchtigkeitsanalysator für pflanzliche Proben, umfassend:
- einen Behälter (2), der die aus einer pflanzlichen Probe (EF) gebildete Ladung aufnimmt und durch eine Antriebsvorrichtung (3) in Drehung versetzt wird,
- eine Einrichtung zum Trocknen (4) durch temperierte trockene Luft, die Blasmittel (43, 44) umfasst, die durch den Behälter (2) auf die Ladung (EF) gerichtet sind,
- eine Einrichtung zum Wiegen (5) des Behälters (2), der die Ladung (EF) enthält,
- eine Steuereinheit (6), die mit dem Behälter (2), der Drehantriebsvorrichtung (3), der Trocknungseinrichtung (4) und der Wiegeeinrichtung (5) verbunden ist, um das Wiegen des beladenen Behälters (2) vor und nach der Trocknungsphase sowie den Drehantrieb des Behälters (2) und die Betätigung der Trocknungseinrichtung (4) zu steuern,
**dadurch gekennzeichnet, dass**
- der Behälter (2) drehbar in einer Halterung (22) montiert ist, die von einer Positionierungseinheit (23) getragen wird, die den Behälter (2) in Eingriff mit der Antriebsvorrichtung (3) bringt, um ihn vor und nach der Drehantriebshase in Drehung zu versetzen oder freizugeben und um ihn in der Wiegeeinrichtung (5) anzuordnen.

2. Feuchtigkeitsanalysator nach Anspruch 1,
**dadurch gekennzeichnet, dass**
der Behälter (2) einen perforierten Korb (21) umfasst, der durchlässig gegenüber den Luftströmen aus den Düsen (43, 44) ist.

3. Feuchtigkeitsanalysator nach Anspruch 1,
**dadurch gekennzeichnet, dass**
die Einheit zur Positionierung des Behälters in Bezug auf die Drehantriebsvorrichtung und die Wiegeeinrichtung ein Hebeorgan ist, das für die Positionierungsvorgänge mit dem Behälter verbunden wird, wobei die Wiegeeinrichtung unter dem Behälter angeordnet ist, damit die Positionierungsvorrichtung durch eine einfache Hebe- und Senkbewegung vorgehen kann.

4. Feuchtigkeitsanalysator nach Anspruch 1,
**dadurch gekennzeichnet, dass**
die Drehantriebsvorrichtung einen Motor und ein Kettenrad umfasst, das direkt mit dem Korb zusammenwirkt, indem es sich an der Seite des Korbs oder an der Drehachse des Korbs dreht.

5. Feuchtigkeitsanalysator nach Anspruch 1,
**dadurch gekennzeichnet, dass**
die Einrichtung zum Trocknen (4) durch temperierte trockene Luft durch eine Quelle für entsprechend eingestellte trockene Druckluft (41) gebildet wird, die eine Verteilungsvorrichtung (42)versorgt, die die Luft auf eine bestimmte Temperatur (T') bringt und gemäß einem Verteilungsmodus (F) mithilfe der Düsen (43, 44) verteilt, die in unterschiedlichen Richtungen (D1, D2) auf die Ladung (EF) gerichtet sind.

6. Feuchtigkeitsanalysator nach Anspruch 5,
**dadurch gekennzeichnet, dass**
die Blasachse (D1) von wenigstens einer der Düsen (43) radial in einer Ebene ausgerichtet ist, die im Wesentlichen senkrecht zur Drehachse (XX) des Korbs (21) angeordnet ist, und die Blasachse (D2) der anderen Düse (44) im Wesentlichen parallel zur Drehachse (XX) ausgerichtet ist.

7. Feuchtigkeitsanalysator nach Anspruch 1,
**dadurch gekennzeichnet, dass**
er ein Gehäuse (1) umfasst, in dem der Behälter (2) und wenigstens die Düsen (43, 44) der Trocknungseinrichtung (4) sowie die Wiegeeinrichtung (5) angeordnet sind, wobei das Gehäuse (4) insbesondere einen Luftabzug (11) umfasst, der die feuchte Luft aus dem Gehäuse (1) entfernt.

8. Feuchtigkeitsanalysator nach Anspruch 1,
**dadurch gekennzeichnet, dass**
die zentrale Einheit (6) ein Mittel zum Aufzeichnen und Anzeigen (8) von Analyseinformationen und ein Mittel zur Eingabe der Parameter (7) umfasst.

9. Feuchtigkeitsanalysator nach Anspruch 1,
**dadurch gekennzeichnet, dass**
die Trocknungseinrichtung ultratrockene Luft mit einem Feuchtigkeitsgehalt zwischen 0 und 5% bei 25°C bereitstellt.

10. Feuchtigkeitsanalysator nach Anspruch 1,
**dadurch gekennzeichnet, dass**
die Trocknungseinrichtung trockene Luft bereitstellt, die auf eine Temperatur von 25°C eingestellt wird.

## Claims

1. Humidity analyser for plant samples, comprising:
- a receiver (2) which receives the load formed by a plant sample (EF) and is driven in rotation by a drive device (3),
- a drying installation (4) for drying by means of heated dry air, having blowing members (43, 44) directed at the load (EF) through the receiver (2),
- a weighing installation (5) for weighing the receiver (2) containing the load (EF),
- a control unit (6) which is connected to the receiver (2), to the device for driving in rotation (3), to the drying installation (4) and to the weighing installation (5) in order to control the weighing of the loaded receiver (2) before and after the drying phase as well as the rotation of the receiver (2) and the actuation of the drying installation (4),
**characterised in that**
- the receiver (2) is rotatably mounted in a support (22) carried by a positioning unit (23) which engages the receiver (2) with the drive device (3) in order to drive it in rotation or to disengage it before and after the phase of driving in rotation and place it in the weighing installation (5).

2. Humidity analyser according to claim 1,
**characterised in that**
the receiver (2) comprises a perforated basket (21) which is permeable to the jets of air from the nozzles (43, 44).

3. Humidity analyser according to claim 1,
**characterised in that**
the unit for positioning the receiver relative to the device for driving in rotation and to the weighing installation is a lifting member connected to the receiver for the positioning operations, the weighing installation being situated beneath the receiver so that the positioning device is able to proceed by a simple lifting and lowering movement.

4. Humidity analyser according to claim 1,
**characterised in that**
the device for driving in rotation comprises a motor and a drive wheel which cooperates directly with the basket by rolling on the side of the basket or on the axis of rotation of the basket.

5. Humidity analyser according to claim 1,
**characterised in that**
the drying installation (4) for drying by means of heated dry air is composed of a source of pressurised dry air (41), regulated, which supplies a distributor (42) for heating it to temperature (T') and distributing it according to a distribution mode (F) with nozzles (43, 44) directed at the load (EF) in different directions (D1, D2).

6. Humidity analyser according to claim 5,
**characterised in that**
at least one of the nozzles (43) has its blowing axis (D1) directed radially in a plane substantially perpendicular to the axis (XX) of rotation of the basket (21), and the other nozzle (44) has its blowing axis (D2) directed substantially parallel to the axis of rotation (XX).

7. Humidity analyser according to claim 1,
**characterised in that**
it comprises an enclosure (1) in which there are housed the receiver (2) and at least the nozzles (43, 44) of the drying installation (4) as well as the weighing installation (5), the enclosure (4) comprises especially an air extractor (11) which removes the humid air from the enclosure (1).

8. Humidity analyser according to claim 1,
**characterised in that**
the central unit (6) comprises a means for recording and displaying (8) analysis information and a means for inputting parameters (7).

9. Humidity analyser according to claim 1,
**characterised in that**
the drying installation supplies ultra-dry air of from 0 to 5% humidity at 25°C.

10. Humidity analyser according to claim 1,
**characterised in that**
the drying installation supplies dry air heated to a regulated temperature of 25°C.
